# EUROPEAN PATENT APPLICATION

(11) **EP 3 701 881 A1**
(43) Date of publication of application: **02.09.2020**
(21) Application number: 20159490.0
(22) Date of filing: 26.02.2020
(51) Int. Cl.: A61B 17/04

(54) **PURSE STRING SUTURE DEVICE**

(30) Priority: 27.02.2019 US 201962810980 P; 14.01.2020 US 202016742302
(71) Applicant: Covidien LP, Mansfield, MA 02048 (US)
(72) Inventor: AVVLN, Srinivasa Murthy Aravalli, 534211 Tanuku (IN)
(74) Representative: Maschio, Antonio

(57) **Abstract**

A surgical applicator is utilized in forming a purse string suture used to, e.g., close internal anal structure or to narrow a passage for performing transanal dissection to create total mesorectal excision. The surgical applicator includes an actuation assembly and a reload operatively coupled with the actuation assembly. The reload includes a sleeve defining a lumen extending therethrough, a drive rod rotatably supported within the sleeve, a surgical fastener including a coil body portion rotatably supported on the drive rod such that rotation of the drive rod advances the surgical fastener, and a suture extending through the drive rod.

## Description

### CROSS-REFERENCE TO RELATED APPLICATION

This application claims the benefit of and priority to U.S. Provisional Patent Application No. 62/810,980 filed February 27, 2019, the entire disclosure of which is incorporated by reference herein.

### BACKGROUND

### Technical Field

The disclosure relates to a surgical device and, more particularly, to a surgical device for forming a purse string suture using surgical fasteners having a helical coil body in conjunction with a suture.

### Background of Related Art

Purse string suture devices are known in the prior art which may include a pair of serrated tissue clamping jaws provided with teeth for clamping the tissue to be sutured therebetween. Such devices include needle passages which extend through the teeth on each jaw for receiving a needle attached to a suture to be threaded through the tissue. In use, the tissue to be sutured is clamped between the jaws and the needle is manually passed through the needle passages in both jaws to thread the suture through the tissue. Thereafter, the jaws are opened and the purse string suture is tightened and wrapped to draw the tissue together. With this type of device, a considerable amount of manual effort and dexterity is required to accomplish the purse string suturing technique. Also, in such devices, it is difficult to control the flow of tissue between the teeth because an insufficient amount of space is provided to gather the tissue clamped by the jaws.

### SUMMARY

In accordance with an embodiment of the disclosure, a surgical applicator for forming a purse string suture includes an actuation assembly and a reload operatively coupled with the actuation assembly. The reload includes a sleeve defining a lumen extending therethrough, a drive rod rotatably supported within the sleeve, a surgical fastener including a coil body portion rotatably supported on the drive rod such that rotation of the drive rod advances the surgical fastener, and a suture extending through the drive rod. The drive rod defines a passage therethrough.

In an embodiment, the drive rod may define a slot configured to slidably engage a portion of the surgical fastener to impart concomitant rotation to the surgical fastener.

In another embodiment, the slot may extend along a length of the drive rod.

In yet another embodiment, the sleeve may have a distal portion having a cutout providing an opening for the surgical fastener to engage tissue.

In still yet another embodiment, the sleeve may include a lateral wall defining the cutout such that a distal tip of the surgical fastener engages tissue while the coil body portion is rotatably supported on the drive rod.

In still yet another embodiment, the sleeve may include an inner wall defining the lumen. The inner wall may include a guide extending radially inward. The guide may be configured to rotatably support the surgical fastener thereon.

In an embodiment, the guide of the inner wall may include a helical coil configuration to facilitate rotational advancement of the surgical fastener.

In another embodiment, the surgical applicator may further include an elongate member detachably supporting the reload thereon. The elongate member may include an actuation shaft operatively coupled to the actuation assembly and the drive rod of the reload for concomitant rotation therewith.

In an embodiment, the drive rod may have a proximal portion defining a cavity having a cross-section complementary to a cross-section of the actuation shaft of the elongate member.

In accordance with another embodiment of the disclosure, a reload for use with a surgical applicator for forming a purse string suture includes a sleeve defining a lumen extending therethrough, a drive rod rotatably supported within the lumen of the sleeve, a plurality of surgical fasteners, and a suture extending through the plurality of surgical fasteners. Each surgical fastener includes a coil body portion supported on the drive rod such that rotation of the drive rod advances the surgical fastener.

In accordance with yet another embodiment of the disclosure, a surgical applicator for forming a purse string suture includes a handle assembly and a reload. The handle assembly includes a motor including an output shaft and an actuator operatively coupled with the motor. The reload is operatively coupled with the motor. The reload includes a sleeve defining a lumen extending therethrough, a drive rod rotatably supported within the sleeve and defining a passage therethrough, a surgical fastener including a coil body portion supported on the drive rod such that rotation of the drive rod advances the surgical fastener, and a suture extending through the passage of the drive rod.

In an embodiment, the handle assembly may further include a battery.

### BRIEF DESCRIPTION OF THE DRAWINGS

Various embodiments of the disclosure are described hereinbelow with reference to the drawings, wherein:
FIG. 1 is a perspective view of a surgical applicator in accordance with an embodiment of the disclosure;
FIG. 2 is a perspective view of a reload of the surgical applicator of FIG. 1 with a portion of a sleeve of the reload removed;
FIG. 3 is another perspective view of a reload of the surgical applicator of FIG. 1, illustrating a plurality of surgical fasteners rotatably supported relative to a helical guide of the sleeve;
FIG. 4 is a perspective view of a drive rod of the reload of FIG. 2, illustrating a plurality of surgical fasteners supported on the drive rod;
FIG. 5 is a perspective view of the reload of FIG. 1 with the sleeve shown in phantom;
FIG. 6 is a cross-sectional view of the reload of FIG. 5 cut along section line 6-6;
FIG. 7 is a partial perspective view of the reload of FIG. 2; and
FIG. 8 is a top view of a surgical site, illustrating use of the surgical applicator of FIG. 1 to form a purse string suture.

### DETAILED DESCRIPTION

Embodiments of the disclosure will now be described in detail with reference to the drawings, in which like reference numerals designate identical or corresponding elements in each of the several views. As used herein, the term "distal," as is conventional, will refer to that portion of the instrument, apparatus, device or component thereof which is farther from the user while, the term "proximal," will refer to that portion of the instrument, apparatus, device or component thereof which is closer to the user. In the following description, well-known functions or constructions are not described in detail to avoid obscuring the disclosure in unnecessary detail.

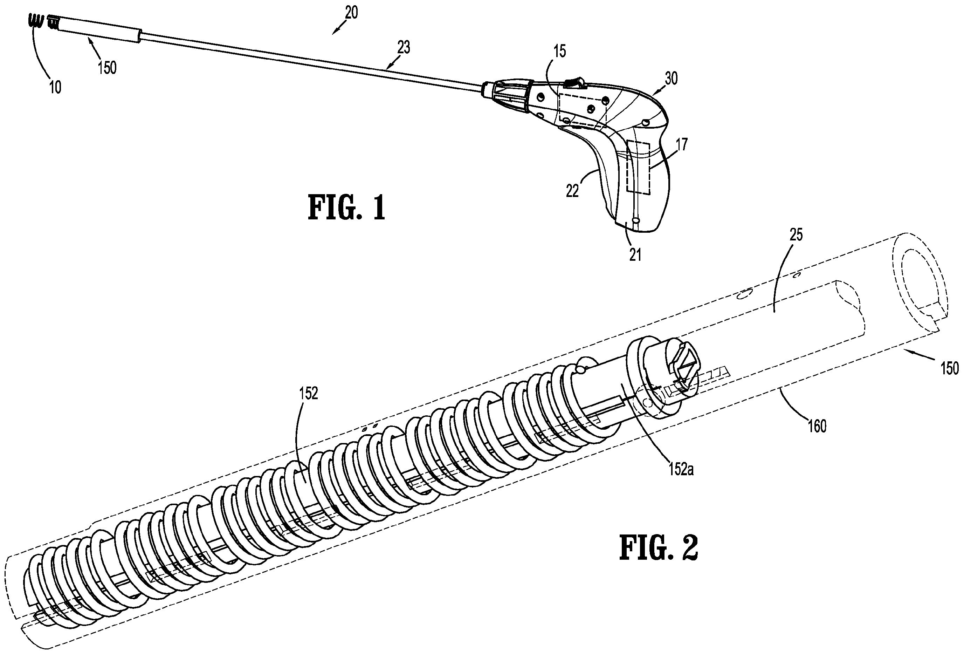

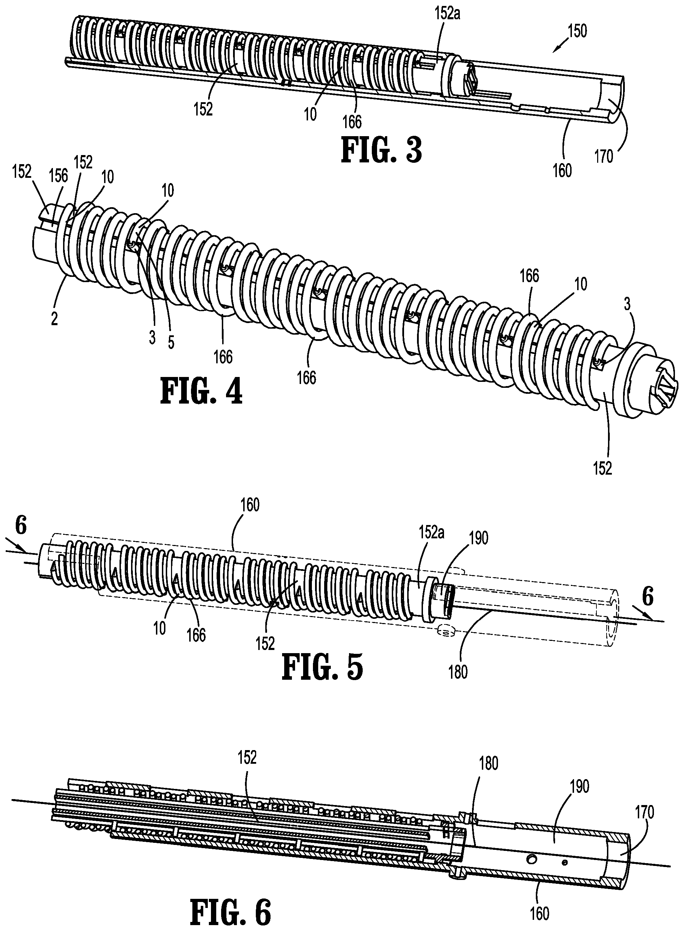

Referring to FIG. 1, there is illustrated a surgical applicator 20 configured to apply surgical fasteners 10 in conjunction with a suture 180 (FIG. 5) to form a purse string suture. For example, the surgical applicator 20 may be utilized in transanal total mesorectal excision (TaTME) for removal of low rectal and ultra-low rectal tumors and preservation of anal sphincters to avoid permanent stomas. In particular, the surgical applicator 20 may be utilized in forming surgical stitch used to close internal anal structure or to narrow a passage for performing further transanal dissection to create total mesorectal excision.

Through the use of the surgical applicator 20, the formation of a purse string suture is simplified by, e.g., eliminating the need for maneuvering a needle inside an anal canal. In addition, uniform needle rotation, tissue penetration, and/or suture advancement may be obtained independent of the skill of the clinician. In this manner, injuries to tissue may be reduced.

The surgical applicator 20 generally includes a handle assembly 30 including a pistol grip handle 21 and an actuator 22, an elongate member 23 extending distally from the handle assembly 30, and a reload 150 releasably coupled to the elongate member 23. An outer diameter of the reload 150 and the elongate member 23 may be dimensioned for use with standard trocars or laparoscopic devices for minimally invasive entry into an opening in tissue. The reload 150 contains a plurality of serially arranged surgical fasteners 10. Actuation of the actuator 22 ejects a surgical fastener 10 out of the reload 150.

Referring to FIGS. 1 and 2, the handle assembly 30 includes a motor 15 (shown in phantom) configured to provide rotational output to a drive rod 152 of the reload 150. The handle assembly 30 may further include, e.g., an internal battery 17 (shown in phantom), to supply power to the motor 15. The actuator 22 is operatively coupled to the motor 15 to actuate the motor 15. The elongate member 23 includes an actuation shaft 25 shown in phantom, rotatably extending through the elongate member 23. In particular, the actuation shaft 25 is coupled with an output shaft (not shown) of the motor 15 and the drive rod 152 of the reload 150.

Referring now to FIGS. 3 and 4, the reload 150 is releasably attached to the elongate member 23 (FIG. 1). In particular, the reload 150 includes a sleeve 160 defining a lumen 170 therethrough, and the drive rod 152 rotatably supported within the lumen 170. In particular, the drive rod 152 is rotatable, but axially stationary. The drive rod 152 may define a passage to receive a suture 180 (FIG. 6) therethrough. The actuation shaft 25 of the elongate member 23 is operatively coupled with the drive rod 152 of the reload 150 for concomitant rotation therewith. For example, the drive rod 152 includes a proximal section 152a configured to operatively engage the actuation shaft 25 (FIG. 2) of the elongate member 23 such that actuation of the motor 15 (FIG. 1) of the handle assembly 30 imparts rotation to the drive rod 52. For example, the proximal section 152a of the drive rod 152 may include a cavity having a cross-section complementary to a cross-section of the actuation shaft 25 such that rotation of actuation shaft 25 causes concomitant rotation of the drive rod 152. In this manner, actuation of the motor 15 of the handle assembly 130 imparts rotation to the drive rod 152.

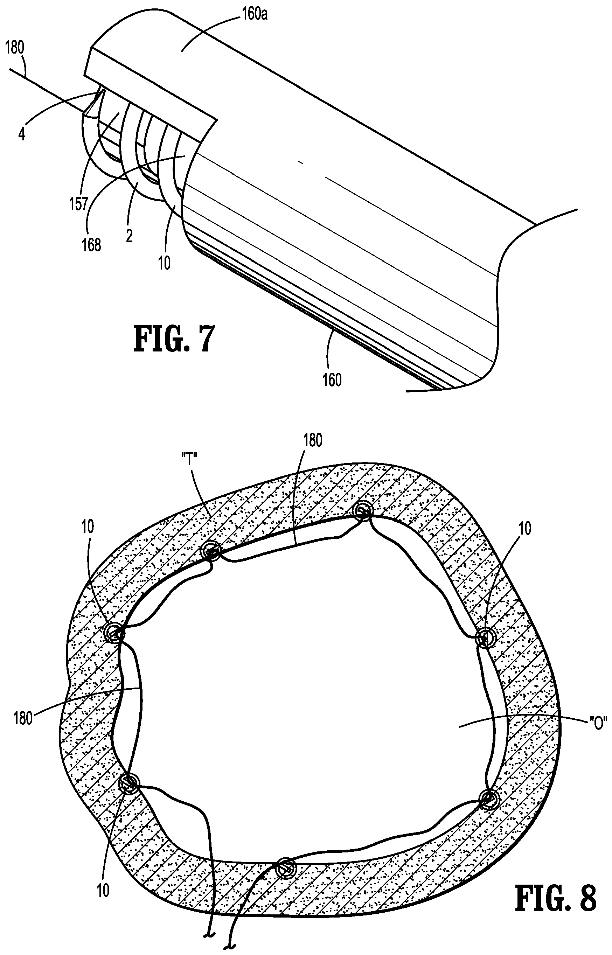

Referring now to FIGS. 4-6, the drive rod 152 has a sufficient length to support multiple surgical fasteners 10 in sequence. In particular, the drive rod 152 defines a slot 156 extending along a length thereof. Each surgical fastener 10 includes a coil body portion 2 terminating in a sharp tissue penetrating point 4 (FIG. 7). A tang 3 is provided at a proximal end 5 of the coil body portion 2. The tang 3 extends generally inwardly toward the center of the coil body portion 2. In particular, the slot 156 of the drive rod 152 is dimensioned to slidably receive the tang 3 of the surgical fastener 10 such that rotation of the drive rod 152 causes concomitant rotation of the surgical fastener 10. It is also contemplated that barbs or a sharp point (not shown) projecting in reverse direction to the sharp tissue penetrating point 4 may be added to enhance anchoring characteristics of the surgical fastener 10.

The coil body portion 2 is in a form of a continuous helix that may be longitudinally collapsible and expandable. The coil body portion 2 has a uniform diameter along a length thereof. However, the coil body portion 2 may be tapered along the length thereof. The pre-formed pitch may be about 0.050 inches. However, the pre-formed pitch may be, e.g., a maximum of approximately 3.0 times a diameter of the coil body portion 2. Alternatively, the pitch may vary along the length of the coil body portion 2 in order to optimize the retaining force of the surgical fastener 10. Moreover, since the coil body portion 2 is longitudinally collapsible and expandable, upon insertion into tissue, the final pitch may be different than the pre-formed pitch. If the coil body portion 2 is made of rigid construction, as is also contemplated, the pitch may be made fixed.

The surgical fastener 10 may be made from semi-stiff implantable wire, such as titanium, wound into a helical shape. Alternatively, the surgical fastener 10 may include plastic or absorbable materials. Examples of materials that can be used in constructing the coil body portion 2 may include titanium, titanium alloys, stainless steel, nickel, chrome alloys and any other biocompatible implantable metals. Alternatively, other options for materials are liquid crystal polymers, HDPE, polyglycolic acid, and polyglycolid hydroxgacetic acid. At least a portion of the coil body portion 2 may be coated with a biocompatible lubricious material that provides for easier delivery of the surgical fastener 10 into tissue.

Referring to FIGS. 4-6, the sleeve 160 includes a helical guide 166 configured to support the surgical fastener 10 and facilitate rotational advancement of the surgical fastener 10 out of the reload 150 when the drive rod 152 is rotated. In particular, the helical guide 166 has a configuration complementary to a configuration of the surgical fastener 10. The reload 150 further includes an adapter portion 190 configured to receive the actuation shaft 25 (FIG. 2) of elongate member 23 operatively coupled to the motor 15 of the handle assembly 30.

Referring to FIG. 7, the sleeve 160 of the reload 150 further includes a distal portion 160a defining a lateral cutout 168 configured to expose the tissue penetrating point 4 of a distal-most surgical fastener 10 to enable the surgical fastener 10 to engage tissue. Under such a configuration, the tissue penetrating point 4 engages tissue while the coil body portion 2 is supported on the drive rod 152, thereby enhancing stability of rotation of the surgical fastener 10 during insertion of the tissue penetrating point 4 into tissue.

Referring now to FIG. 8, in use, the surgical applicator 20 containing the surgical fasteners 10 is positioned adjacent the opening "O" in tissue "T" such that the surgical fasteners 10 may be applied to peripheral portions of the opening "O" in tissue "T." Thereafter, the actuator 22 is actuated to cause rotation of the drive rod 152 relative to the sleeve 160 which, in turn, drives the surgical fastener 10 through a distal opening 157 (FIG. 7) of the reload 150 and into tissue "T". The clinician repeats the process until there are sufficient number of the surgical fasteners 10 surrounding the opening "O" of tissue "T" to provide stable anchoring when opposite ends of the suture 180 (FIG. 7) are pulled to close the opening "O." Once the plurality of surgical fasteners 10 are anchored on a peripheral portion of the opening "O," the opposing ends of the suture 180 are pulled by the clinician to close the opening "O." In this manner, the formation of a purse string suture is simplified by, e.g., eliminating the need for maneuvering a needle inside an anal canal, which may reduce injuries to tissue.

Although the illustrative embodiments of the disclosure have been described herein with reference to the accompanying drawings, the above description, disclosure, and figures should not be construed as limiting, but merely as exemplifications of particular embodiments. For example, while a powered actuation utilizing a motor has been described in this disclosure, it is also envisioned that a mechanically triggered actuation assembly may be utilized to provide rotational output to the drive rod 152 of the reload 150. It is further contemplated that the surgical applicator 20 may be adapted for use in robotic surgery.

It is also to be appreciated that the disclosure may be utilized in a number of applications including ligating tissue, hernia mesh repair, and in conjunction with implant drug delivery systems or procedures involving positioning of surgical or implantable devices in patients. It is to be understood, therefore, that the disclosure is not limited to those precise embodiments, and that various other changes and modifications may be effected therein by one skilled in the art without departing from the scope or spirit of the disclosure.

The present invention may be described as the following numbered paragraphs:-
1. A surgical applicator for forming a purse string suture comprising: an actuation assembly; and a reload operatively coupled with the actuation assembly, the reload including: a sleeve defining a lumen extending therethrough; a drive rod rotatably supported within the sleeve, the drive rod defining a passage therethrough; a surgical fastener including a coil body portion rotatably supported on the drive rod such that rotation of the drive rod advances the surgical fastener; and a suture extending through the passage of the drive rod.
2. The surgical applicator according to paragraph 1, wherein the drive rod defines a slot configured to slidably engage a portion of the surgical fastener to impart concomitant rotation to the surgical fastener.
3. The surgical applicator according to paragraph 2, wherein the slot extends along a length of the drive rod.
4. The surgical applicator according to paragraph 1, wherein the sleeve has a distal portion having a cutout providing an opening for the surgical fastener to engage tissue.
5. The surgical applicator according to paragraph 4, wherein the sleeve includes a lateral wall defining the cutout such that a distal tip of the surgical fastener engages tissue while the coil body portion is rotatably supported on the drive rod.
6. The surgical applicator according to paragraph 1, wherein the sleeve includes an inner wall defining the lumen, the inner wall includes a guide extending radially inward, and the guide is configured to rotatably support the surgical fastener thereon.
7. The surgical applicator according to paragraph 6, wherein the guide of the inner wall includes a helical coil configuration configured to facilitate rotational advancement of the surgical fastener.
8. The surgical applicator according to paragraph 1, further including an elongate member detachably supporting the reload thereon, the elongate member including an actuation shaft operatively coupled to the actuation assembly and the drive rod of the reload.
9. The surgical applicator according to paragraph 8, wherein the drive rod has a proximal portion defining a cavity having a cross-section complementary to a cross-section of the actuation shaft of the elongate member.
10. A reload for use with a surgical applicator for forming a purse string suture, comprising: a sleeve defining a lumen extending therethrough; a drive rod rotatably supported within the lumen of the sleeve, the drive rod defining a passage therethrough; a plurality of surgical fasteners, each surgical fastener of the plurality of surgical fasteners including a coil body portion supported on the drive rod such that rotation of the drive rod advances the plurality of surgical fasteners; and a suture extending through the plurality of surgical fasteners.
11. The reload according to paragraph 10, wherein the drive rod defines a longitudinal slot configured to engage a portion of each surgical fastener of the plurality of surgical fasteners to cause concomitant rotation with the plurality of surgical fasteners.
12. The reload according to paragraph 11, wherein each surgical fastener of the plurality of surgical fasteners includes a tang extending radially inward from the coil body portion, the tang configured to slidably engage the longitudinal slot of the drive rod.
13. The reload according to paragraph 10, wherein the sleeve has a distal portion having a cutout providing a lateral opening for a distal-most surgical fastener of the plurality of surgical fasteners to engage tissue.
14. The reload according to paragraph 10, wherein the sleeve includes an inner wall defining the lumen, the inner wall includes a guide extending radially inward, and the guide is configured to rotatably support the plurality of surgical fasteners thereon.
15. The reload according to paragraph 14, wherein the guide of the inner wall includes a plurality of helical coils configured to facilitate rotational advancement of the surgical fastener.
6. A surgical applicator for forming a purse string suture, comprising: a handle assembly including: a motor including an output shaft; and an actuator operatively coupled with the motor; and a reload operatively coupled with the motor, the reload including: a sleeve defining a lumen extending therethrough; a drive rod rotatably supported within the sleeve and defining a passage therethrough; a surgical fastener including a coil body portion rotatably supported on the drive rod such that rotation of the drive rod advances the surgical fastener; and a suture extending through the passage of the drive rod.
17. The surgical applicator according to paragraph 16, wherein the sleeve has a distal portion having a cutout providing a lateral opening for the surgical fastener to engage tissue.
18. The surgical applicator according to paragraph 16, wherein the sleeve includes an inner wall defining the lumen, the inner wall includes a guide extending radially inward, and the guide is configured to rotatably support the surgical fastener thereon.
19. The surgical applicator according to paragraph 18, wherein the guide of the inner wall includes a plurality of helical coils arranged along a length of the reload.
20. The surgical applicator according to paragraph 16, wherein the handle assembly further includes a battery.

## Claims

1. A surgical applicator for forming a purse string suture comprising:
an actuation assembly; and
a reload operatively coupled with the actuation assembly, the reload including:
a sleeve defining a lumen extending therethrough;
a drive rod rotatably supported within the sleeve, the drive rod defining a passage therethrough;
a surgical fastener including a coil body portion rotatably supported on the drive rod such that rotation of the drive rod advances the surgical fastener; and
a suture extending through the passage of the drive rod.

2. The surgical applicator according to claim 1, wherein the drive rod defines a slot configured to slidably engage a portion of the surgical fastener to impart concomitant rotation to the surgical fastener.

3. The surgical applicator according to claim 2, wherein the slot extends along a length of the drive rod.

4. The surgical applicator according to any preceding claim, wherein the sleeve has a distal portion having a cutout providing an opening for the surgical fastener to engage tissue; preferably wherein the sleeve includes a lateral wall defining the cutout such that a distal tip of the surgical fastener engages tissue while the coil body portion is rotatably supported on the drive rod.

5. The surgical applicator according to any preceding claim, wherein the sleeve includes an inner wall defining the lumen, the inner wall includes a guide extending radially inward, and the guide is configured to rotatably support the surgical fastener thereon; preferably wherein the guide of the inner wall includes a helical coil configuration configured to facilitate rotational advancement of the surgical fastener.

6. The surgical applicator according to any preceding claim, further including an elongate member detachably supporting the reload thereon, the elongate member including an actuation shaft operatively coupled to the actuation assembly and the drive rod of the reload; preferably wherein the drive rod has a proximal portion defining a cavity having a cross-section complementary to a cross-section of the actuation shaft of the elongate member.

7. A reload for use with a surgical applicator for forming a purse string suture, comprising:
a sleeve defining a lumen extending therethrough;
a drive rod rotatably supported within the lumen of the sleeve, the drive rod defining a passage therethrough;
a plurality of surgical fasteners, each surgical fastener of the plurality of surgical fasteners including a coil body portion supported on the drive rod such that rotation of the drive rod advances the plurality of surgical fasteners; and
a suture extending through the plurality of surgical fasteners.

8. The reload according to claim 7, wherein the drive rod defines a longitudinal slot configured to engage a portion of each surgical fastener of the plurality of surgical fasteners to cause concomitant rotation with the plurality of surgical fasteners; preferably wherein each surgical fastener of the plurality of surgical fasteners includes a tang extending radially inward from the coil body portion, the tang configured to slidably engage the longitudinal slot of the drive rod.

9. The reload according to claim 7 or claim 8, wherein the sleeve has a distal portion having a cutout providing a lateral opening for a distal-most surgical fastener of the plurality of surgical fasteners to engage tissue.

10. The reload according to any of claims 7 to 9, wherein the sleeve includes an inner wall defining the lumen, the inner wall includes a guide extending radially inward, and the guide is configured to rotatably support the plurality of surgical fasteners thereon; preferably wherein the guide of the inner wall includes a plurality of helical coils configured to facilitate rotational advancement of the surgical fastener.

11. A surgical applicator for forming a purse string suture, comprising:
a handle assembly including:
a motor including an output shaft; and
an actuator operatively coupled with the motor; and
a reload operatively coupled with the motor, the reload including:
a sleeve defining a lumen extending therethrough;
a drive rod rotatably supported within the sleeve and defining a passage therethrough;
a surgical fastener including a coil body portion rotatably supported on the drive rod such that rotation of the drive rod advances the surgical fastener; and
a suture extending through the passage of the drive rod.

12. The surgical applicator according to claim 11, wherein the sleeve has a distal portion having a cutout providing a lateral opening for the surgical fastener to engage tissue.

13. The surgical applicator according to claim 11 or claim 12, wherein the sleeve includes an inner wall defining the lumen, the inner wall includes a guide extending radially inward, and the guide is configured to rotatably support the surgical fastener thereon.

14. The surgical applicator according to claim 13, wherein the guide of the inner wall includes a plurality of helical coils arranged along a length of the reload.

15. The surgical applicator according to any of claims 11 to 14, wherein the handle assembly further includes a battery.
